# EUROPÄISCHE PATENTSCHRIFT

(11) **EP 1 453 527 B1**
(45) Veröffentlichungstag und Bekanntmachung des Hinweises auf die Patenterteilung: **13.09.2006**
(21) Anmeldenummer: 02787839.6
(22) Anmeldetag: 27.11.2002
(51) Int. Cl.: A61K 35/22, A61K 38/22, C07J 75/00

(54) **VERFAHREN ZUR GEWINNUNG VON OESTROGENEN AUS STUTENHARN**
METHOD FOR OBTAINING OESTROGEN FROM MARE URINE
PROCEDE D'OBTENTION D'ESTROGENES A PARTIR D'URINE DE JUMENT

(30) Priorität: 01.12.2001 DE 10159161
(43) Veröffentlichungstag der Anmeldung: 08.09.2004
(73) Patentinhaber: Solvay Pharmaceuticals GmbH, 30173 Hannover (DE)
(72) Erfinder: RASCHE, Heinz-Helmer, 31303 Burgdorf (DE); RUPP, Olaf, 15566 Schöneiche bei Berlin (DE)
(74) Vertreter: Gosmann, Martin
(86) Internationale Anmeldenummer: PCT/EP2002/013343
(87) Internationale Veröffentlichungsnummer: WO 2003/048183

(56) Entgegenhaltungen:
- WO-A-98/08525
- US-A- 5 723 454
- BRADLOW H L: "EXTRACTION OF STEROID CONJUGATES WITH A NEUTRAL RESIN" STEROIDS, ELSEVIER SCIENCE PUBLISHERS, NEW YORK, NY, US, Bd. 11, Nr. 3, 1. März 1968 (1968-03-01), Seiten 265-272, XP002030905 ISSN: 0039-128X

## Beschreibung

Die vorliegende Erfindung betrifft die Gewinnung eines natürlichen Gemisches konjugierter Oestrogene aus dem Harn trächtiger Stuten.

Oestrogene werden in der Medizin zur Hormonsubstitutionstherapie eingesetzt. Insbesondere werden Oestrogengemische zur Behandlung und Prophylaxe der bei Frauen auftretenden Beschwerden der Wechseljahre nach natürlicher oder artifizieller Menopause eingesetzt. Hierbei haben sich natürliche Gemische konjugierter Oestrogene, wie sie im Harn trächtiger Stuten vorliegen, als besonders wirksam und gut verträglich erwiesen.

Der gelöste Feststoffgehalt im Harn trächtiger Stuten (= pregnant mares urine, im folgenden abgekürzt als "PMU") kann natürlicherweise in weiten Bereichen schwanken und im allgemeinen in einem Bereich von 40 bis 90 g Trockensubstanz pro Liter liegen. Neben Harnstoff und sonstigen üblichen Harninhaltsstoffen sind in dem Feststoffgehalt des PMU phenolische Bestandteile in Mengen von ca. 2 bis 5 Gew.-% bezogen auf die Trockensubstanz enthalten. Unter diesen phenolischen Bestandteilen befinden sich Kresole und das als HPMF bekannte Dihydro-3,4-bis[(3-hydroxyphenyl)methyl]-2(3H)-furanon. Diese können in freier oder konjugierter Form vorliegen. In dem PMU ist ein natürliches Gemisch von Oestrogenen enthalten, welches weitgehend in konjugierter Form, z. B. als Schwefelsäurehalbester-Natriumsalz (im folgenden abgekürzt als "Sulfatsalz"), vorliegt. Der Gehalt an konjugierten Oestrogenen (berechnet als Oestrogensulfatsalz) kann zwischen 0,3 und 1 Gew.-% bezogen auf die Trockensubstanz betragen.

Aus der WO 98/08526 ist bereits ein geeignetes Verfahren bekannt, mit welchem in einer Festphasen-Extraktion an einem semipolaren, insbesondere nichtionischen semipolaren, polymeren Adsorberharz ein an phenolischen Harninhaltsstoffen abgereichertes, weitgehend Kresol- und HPMF-freies, den natürlichen Oestrogen-Gehalt des PMU praktisch vollständig enthaltendes Gemisch gewonnen werden kann. Dieser Extrakt eignet sich als Ausgangsmaterial für die Herstellung von Pharmazeutika, die das natürliche Gemisch konjugierter Oestrogene als Wirkstoffkomponente enthalten.

Um als Wirkstoffkomponente für Pharmazeutika verwendet werden zu können, muss das gewonnene natürliche Gemisch konjugierter Oestrogene jedoch bestimmte pharmazeutische Spezifikationen erfüllen, z.B. der in der USP (United States Pharmacopeia) oder European Pharmacopeia festgelegten Spezifikation entsprechen. Auch sollte der Gehalt an freien Oestrogenen bezogen auf die Trockensubstanz bestimmte Grenzwerte nicht übersteigen. Normalerweise werden die gestellten pharmazeutischen Spezifikationsanforderungen von den aus PMU gemäß dem Verfahren der WO 98/08526 gewonnenen Gemischen konjugierter Oestrogene erfüllt.

Es hat sich aber herausgestellt, dass die geforderten pharmazeutischen Spezifikationen, beispielsweise der vorstehende Grenzwert des maximal tolerierbaren Gehaltes an freien Oestrogenen, oft dann nicht eingehalten werden kann, wenn ein "gealteter" Harn, z.B. ein über längere Zeit und/oder ggf. bei erhöhten Temperaturen gelagerter oder transportierter PMU, zur Gewinnung des natürlichen Gemisches konjugierter Oestrogene verwendet wird. Diese Alterung des PMU ist wahrscheinlich darauf zurückzuführen, dass je nach Lager- und/oder Transportbedingungen der Gehalt an konjugierten Oestrogenen mit der Zeit oder bei erhöhten Temperaturen abnehmen kann, während der Gehalt an unerwünschten freien Oestrogenen in der Folge zunimmt. Die ursprüngliche Zusammensetzung des natürlichen Gemisches an Oestrogenen kann somit gegenüber frischem Harn bei gealtertem Harn nachteilig verändert sein. Für das mit dem Verfahren der WO 98/08526 gewonnene natürliche Gemisch konjugierter Oestrogene besteht bei gegebenfalls veränderter Zusammensetzung eines gealterten Ausgangsproduktes oft die Gefahr, dass der gewonnene Extrakt nicht mehr die geforderten pharmazeutischen Spezifikationen, insbesondere den maximal tolerierbaren Gehalt an freien Oestrogenen einhalten kann. Besonders nachteilig daran ist, dass aus gealtertem PMU gewonnene Chargen mit dem an sich wertvollen Wirkstoff-Extrakt des Gemisches konjugierter Oestrogene dann nicht mehr zur Herstellung eines pharmazeutischen Präparates verwendet werden kann und daher verworfen werden muss. Der Verlust eines wertvollen natürlichen pharmazeutischen Wirkstoffes ist angesichts der aufwendigen und nur zu bestimmten Perioden der Trächtigkeit durchführbaren Sammlung von PMU und der damit verbundenen logistischen Randbedingungen in der Praxis und unter wirtschaftlichen Gesichtspunkten in hohem Maße unbefriedigend.

Aufgabe der vorliegenden Erfindung ist es daher, ein technisch und wirtschaftlich optimales Verfahren zur Gewinnung eines an phenolischen Harninhaltsstoffen abgereicherten natürlichen Gemisches konjugierter Oestrogene aus dem PMU zu entwickeln, bei dem als Ausgangsprodukt auch gealterter PMU, d.h. über längere Zeiträume und/oder bei erhöhten Temperaturen gelagerter oder transportierter PMU, der ggf. einen erhöhten Anteil an freien Oestrogenen aufweisen kann, sicher ohne Ausschuß verwendet werden kann, und bei dem insbesondere das aus diesem PMU gewonnene natürliche Gemisch konjugierter Oestrogene gute Wirkstoffgehalte aufweist und die geforderten pharmazeutische Spezifikation, insbesondere auch im Hinblick auf den maximal tolerierbaren Gehalt an freien Oestrogenen, erfüllt.

Es wurde nun ein Verfahren gefunden, mit welchem auf überraschend einfache Weise ein Gemisch konjugierter Oestrogene auch aus gealtertem, d.h. insbesondere aus einem ggf. einen erhöhten Anteil an freien Oestrogenen aufweisenden, PMU gewonnenen werden kann, wobei das gewonnene Gemisch konjugierter Oestrogene eine hohe Produktqualität aufweist und die Anforderungen pharmazeutischer Spezifikation, insbesondere auch im Hinblick auf den maximal tolerierbaren Gehalt an freien Oestrogenen, sicher erfüllt.

Das erfindungsgemäße Verfahren geht aus von dem in der WO 98/08526 beschriebenen Verfahren, welches zur Gewinnung eines an phenolischen Harninhaltsstoffen abgereicherten natürlichen Gemisches konjugierter Oestrogene aus dem PMU dient. Demgemäß betrifft die Erfindung ein Verfahren zur Gewinnung eines an phenolischen Harninhaltsstoffen abgereicherten natürlichen Gemisches konjugierter Oestrogene aus dem Harn trächtiger Stuten bei dem man
a) eine Harnflüssigkeit, welche gegebenenfalls eine von Schleim- und Feststoffen befreite Harnfüssigkeit, ein eingeengtes Konzentrat dieser Harnflüssigkeit oder ein durch Membranfiltration dieser Harnflüssigkeit erhaltenes eingeengtes Harnretentat darstellt, mit einer zur Adsorption des in der Harnflüssigkeit enthaltenen Gemisches konjugierter Oestrogene ausreichenden Menge eines semipolaren polymeren Adsorberharzes kontaktiert und ein mit dem Gemisch konjugierter Oestrogene beladenes semipolares polymeres Adsorberharz von der übrigen Harnflüssigkeit abtrennt,
b) das mit dem Gemisch konjugierter Oestrogene beladene semipolare polymere Adsorberharz mit einem auf einen pH-Bereich von mindestens 12,0 insbesondere von 12,5 bis 14,0, eingestellten Waschwasser wäscht
c) das gewaschene Adsorberharz mit einer zur Desorption des daran adsorbierten Gemisches konjugierter Oestrogene ausreichenden Menge einer Elutionsflüssigkeit, welche ein mit Wasser mischbares organisches Lösemittel aus der Gruppe mit Wasser mischbarer Ether, niederer Alkanole und niederer aliphatischer Ketone oder ein Gemisch aus dem mit Wasser mischbaren organischen Lösemittel und gegebenenfalls alkalisch eingestelltem Wasser darstellt, kontaktiert und ein das natürliche Gemisch konjugierter Oestrogene enthaltendes Eluat von dem Adsorberharz abtrennt und gegebenenfalls einengt, wobei sich das erfindungsgemäße Verfahren gegenüber dem Verfahren des Standes der Technik dadurch auszeichnet, dass

man zwischen den Verfahrensschritten b) und c) eine Zwischenwäsche durchführt, bei der das mit dem Gemisch konjugierter Oestrogene beladene semipolare Adsorberharz mit Wasser gewaschen wird.

Die Chargenvorbereitung, die an sich bekannten Verfahrensschritte a), b) und c) sowie die Verwendung des im Verfahrensschritt c) erhaltenen Eluats, das ein Gemisch natürlicher konjugierter Oestrogene enthält, sind generell bereits in der internationalen Patentanmeldung WO 98/08526 beschrieben und dem Fachmann somit aus dieser publizierten Patentanmeldung geläufig. Der Inhalt dieser WO 98/08526 wird zum Zwecke der Offenbarung auch zum Gegenstand der vorliegenden Anmeldung gemacht. Weitere Einzelheiten zur allgemeinen Verfahrensweise und einsetzbaren Materialien sind im Beispielteil der vorliegenden Anmeldung zusammengefaßt. Beispielsweise können gemäß WO 98/08526 semipolare, insbesondere nichtionische semipolare, Adsorberharze eingesetzt werden. Darüber hinaus ist es gemäß dem Verfahren der vorliegenden Erfindung überraschenderweise auch möglich andere Adsorberharze zusammen mit der erfindungsgemäßen Zwischenwäsche einzusetzen, ohne dass die Produktqualität bzw. die einzuhaltende pharmazeutische Spezifikation beeinträchtigt wird. Die im Rahmen der vorliegenden Erfindung verwendbaren Adsorberharze werden weiter unten in der Beschreibung noch näher erläutert.

Erfindungsgemäß wird das im Verfahrensschnitt a) mit dem Gemisch konjugierter Oestrogene beladene semipolare Adsorberharz in einer sich an den Verfahrensschritt b) anschließenden Zwischenwäsche mit Wasser gewaschen. Die Menge an Waschwasser wird so gewählt, dass das im anschließenden Verfahrensschritt c) erhaltene Eluat ein Gemisch konjugierter Oestrogene aufweist, das die Anforderungen an einen Höchstgehalt an freien Oestrogenen erfüllt und damit als Wirkstoffkomponente für Pharmazeutika verwendet werden kann. Als zweckmäßig erweist sich beispielsweise die Verwendung von 2 bis 8, vorzugsweise 3 bis 5, Bettvolumen Waschwasser pro Bettvolumen Adsorberharz. Hierbei wird das Waschwasser zweckmäßigerweise durch einen das Adsorberharz enthaltenden Reaktor mit einer Durchflussgeschwindigkeit von 3 bis 10, vorzugsweise 5 bis 7, Vol.-Teilen Waschwasser pro 1 Vol.-Teil Adsorberharz pro Stunde geleitet.

Es muss als ausgesprochen überraschend bezeichnet werden, dass ein vermeintliches einfaches, zwischen den Verfahrensschritten b) und c) durchgeführtes Waschen des Adsorberharzes mit Wasser auch bei Verwendung von gealtertem PMU derart zur Optimierung der pharmazeutischen Spezifikation der als Wirkstoff-Extrakt gewonnenen natürlichen Gemische konjugierter Oestrogene führt, wie erfindungsgemäß festgestellt wurde. Insbesondere ist es sehr überraschend, dass der vor allem in gealtertem PMU gegebenenfalls enthaltene erhöhte Anteil an freien Oestrogenen derart sicher verringert werden kann, dass im Verfahrensschritt c) als Eluat ein Gemisch natürlicher konjugierter Oestrogene erhalten werden kann, welches die hohen Anforderungen an die pharmazeutische Spezifikation, beispielsweise die gemäß der USP oder der Europäischen Pharmacopeia aufgestellten Anforderungen, erfüllt und insbesondere auch aus gealtertem PMU ein natürliche konjugierte Oestrogen-Gemische enthaltendes qualitativ und quantitativ hohwertiges Wirkstoff-Extrakt erhalten werden kann. Diese Wirkstoff-Extrakte halten die maximal tolerierbaren Höchstgehalte an freien Oestrogenen unabhängig von Alter und Herkunft des eingesetzten PMU sicher ein und erweisen sich somit als ein vorteilhaftes bzw. wertvolles Ausgangsprodukt für die Herstellung von Pharmazeutika. Ein unerwünschter und wirtschaftlich nachteiliger Verlust von wertvollen pharmazeutischen Rohstoffen, wie hier die Gemische aus natürlichen konjugierten Oestrogenen, kann somit auch unter schwierigen Bedingungen vermieden werden.

Es hat sich beim erfindungsgemäßen Verfahren als weiterer Vorteil herausgestellt, dass das im Verfahrensschritt c) gewonnene Eluat im Vergleich zum Stand der Technik einen, bezogen auf die Trockensubstanz, erhöhten Gesamthormongehalt aufweist. Dadurch wird ein z.B. in bezug auf den WirkstoffGehalt deutlich verbessertes Qualitätsprodukt erhalten.

In einer vorteilhaften Ausgestaltung des erfindungsgemäßen Verfahrens wird die Zwischenwäsche bei Temperaturen unterhalb Raumtemperatur, insbesondere bei Temperaturen zwischen 0 °C und 10 °C, durchgeführt, da sich gezeigt hat, dass durch die zusätzliche Zwischenwäsche gegebenenfalls bedingte Hormon- bzw. Wirkstoffverluste deutlich vermindert werden können. Als Raumtemperatur wird üblicherweise die Umgebungstemperatur angesehen, z.B. eine Temperatur bezeichnet, die zwischen 20° und 30°C liegt. Sehr zweckmäßig ist es, das Verfahren bei Temperaturen von tatsächlich 0 °C bzw. annähernd 0 °C auszuführen. In der Praxis empfiehlt es sich daher bei Temperaturen von nahe oberhalb 0 °C zu arbeiten und durch entsprechende Maßnahmen für die Einhaltung der genannten Temperaturbereiche zu sorgen. Hierzu können an sich übliche Maßnahmen zur Absenkung der Temperatur getroffen werden, z.B. der Einsatz gekühlter Reaktoren, gekühlter Materialien und/oder gekühlter Ausgangsstoffe wie PMU. Als Temperaturen von 0 °C bzw. von annähernd 0 °C kann unter praktischen Gesichtspunkten ein Temperaturbereich von 0 °C bis etwa 5 °C, insbesondere von 0 °C bis etwa 3 °C, betrachtet werden.

Um mögliche Hormonverluste während der Zwischenwäsche so gering wie möglich zu halten, wird gemäß dieser Variante der Erfindung das in der Zwischenwäsche und/oder auch das im Verfahrensschritt b) verwendete alkalisch gestellte Waschwasser auf Temperaturen unterhalb Raumtemperatur, insbesondere auf Temperaturen zwischen 0 °C und 10 °C, vorgekühlt sein. Weitere zweckmäßige bzw. bevorzugte Temperaturbereiche sind wie vorstehend angegeben Temperaturen von 0 °C bis etwa 5 °C, insbesondere von 0 °C bis etwa 3 °C. Bevorzugt wird bei Temperaturen von 0 °C bzw. von annähernd 0 °C gearbeitet, d.h. vorzugsweise ist das in der Zwischenwäsche und/oder auch das im Verfahrensschritt b) verwendete alkalisch gestellte Waschwasser auf Temperaturen nahe oberhalb 0 °C vorgekühlt. Durch die Verwendung von gekühltem alkalisch gestelltem Waschwasser im Verfahrensschritt b) wird eine Art Vorkühlung oder Aufrechterhaltung der bereits erfolgten Kühlung des Adsorberharzes erreicht, z.B. um zu vermeiden, dass beim Einsatz von gekühlten Waschwasser für die Zwischenwäsche eine unerwünschte Wiedererwärmung desselben stattfindet. Bevorzugt werden daher die Zwischenwäsche und der vorhergehende Verfahrensschritt b) beide im selben Temperaturbereich durchgeführt, z.B. bei Temperaturen unterhalb Raumtemperatur, insbesondere bei Temperaturen zwischen 0 °C und 10 °C, oder vorzugsweise in den wie oben angegebenen Temperaturbereichen.

In der vorstehenden Variante der Erfindung, in der das Verfahren bei Temperaturen unterhalb Raumtemperatur ausgeführt wird, kann es wünschenswert sein, alle verwendeten Vorrichtungen wie Reaktoren zur Aufnahme des semipolaren Adsorberharzes oder dieses bereits enthaltende Reaktoren und/oder den eingesetzten PMU entsprechend auf Temperaturen unterhalb Raumtemperatur, insbesondere auf Temperaturen zwischen 0 °C und 10 °C, oder auf die oben angegebenen vorzugsweisen Temperaturbereiche vorgekühlt einzusetzen.

Als Adsorbens für das erfindungsgemäße Verfahren sind polymere Adsorberharze für den Einsatz geeignet. Besonders bevorzugte Adsorberharze sind semipolare, insbesondere nicht-ionische semipolare, polymere Adsorberharze. Die im erfindungsgemäßen Verfahren als Adsorbens einsetzbaren semipolaren polymeren Adsorberharze sind vorzugsweise poröse organische nichtionische Polymere, welche im Gegensatz zu unpolaren hydrophoben polymeren Adsorberharzen eine intermediäre Polarität (= z.B. mit einem Dipolmoment der aktiven Oberfläche des Harzes im Bereich von 1,0 bis 3,0, insbesondere 1,5 bis 2,0, Debye) und eine etwas hydrophylere Struktur besitzen, beispielsweise Polycarbonsäureesterharze. Zweckmäßig werden makroporöse semipolare Harze mit vorzugsweise makroretikularer Struktur und mit durchschnittlichen Porendurchmessern im Bereich von 50 bis 150, vorzugsweise 70 bis 100, Angström und einer spezifischen Oberfläche im Bereich von 300 bis 900, vorzugsweise 400 bis 500, m²/g eingesetzt. Als besonders geeignet erweisen sich makroporöse quervernetzte aliphatische Polycarbonsäureesterharze, insbesondere quervernetzte Polyacrylesterharze wie z.B. Amberlite XAD-7^{R} der Fa. Rohm und Haas, die nicht-ionische semipolare Adsorberharze darstellen.

Neben den bevorzugt genannten Adsorbenzien können auch andere Adsorberharze eingesetzt werden. Als Adsorberharze eignen sich dabei sowohl unpolare, semipolare und auch polare Adsorberharze. Die Menge des Harns, die über den Adsorber gepumpt werden kann, ist hierbei zuvor anhand der jeweiligen Adsorberkapazität zu bestimmen. Beispiele für verwendbare Adsorberharze sind handelsübliche Typen wie polymere Amberlite Adsorbenzien mit Styroldivinylbenzolgrundgerüsten (z.B. Typen XAD-1180, XAD-2, XAD-4, XAD-16), mit Acrylestergrundgerüsten (z.B. XAD-7) oder solche mit hochpolaren Grundgerüsten enthaltend Stickstoff und Sauerstoff (z.B. XAD-12). Andere Adsorberharze sind Dowex-Harze (Copolymere von Styrol und Divinylbenzol), wie z.B. Dowex 112, Dowex Optipore, Dowex Optipore V 493; Lewatite (vernetzte Polystyrole), z.B. Lewatit OC 1064, Lewatit OC 1066 oder Lewatit OC 1163, Polyaminanionentauscherharze, z.B. Dowex-Harze. Vorteilhafte Adsorberharze sind insbesondere XAD-7, XAD-16 (Typ HP), XAD 118 und Dowex Optipore, bevorzugt als Dowex Optipore V 493, sowie Lewatite OC 1064, OC 1066 und OC 1163.

Das erfindungsgemäße Verfahren bietet, wie vorstehend bereits im Detail beschrieben, eine Reihe von Vorteilen und Verbesserungen gegenüber dem Stand der Technik. So ermöglicht die Erfindung die Verwendung auch von gealtertem PMU, der auch einen erhöhten Anteil an freien Oestrogenen aufweisen darf, ohne dass einzuhaltende pharmazeutische Spezifikationen gefährdet werden. Das erfindungsgemäße Verfahren weist daher auch wirtschaftliche Vorteile auf, da die Gefahr wertvolle Wirkstoffe bei Nichteinhaltung der pharmazeutischen Spezifikation, z.B. bei nicht mehr tolerierbaren Gehalten an freien Oestrogenen, zu verlieren, deutlich vermindert ist. Das erfindungsgemäße Verfahren liefert eine qualitätsverbesserte Wirkstoffkomponente mit einem auf den Trockensubstanz-Gehalt bezogenen erhöhtem Hormongehalt. Diese Wirkstoffkomponente eignet sich hervorragend für die Herstellung von Pharmazeutika, die als Wirkstoff ein Gemisch natürlicher konjugierter Oestrogene enthalten.

Die nachfolgenden Beispiele sollen die Erfindung näher erläutern, ohne jedoch ihren Umfang zu beschränken.

### Beispiele

In den nachfolgenden Beispielen werden allgemeine Arbeitsvorschriften zur Gewinnung von Wirkstoff-Extrakten aus PMU angegeben, die das natürliche Gemisch der im PMU enthaltenen konjugierten Oestrogene enthalten und an phenolischen Harninhaltsstoffen weitgehend abgereichert sind. Es wird gezeigt, wie sich erfindungsgemäß auch aus gealtertem PMU, der einen erhöhten Anteil an freien Oestrogenen aufweisen kann, ein Qualitätsextrakt mit hohen Wirkstoffgehalten gewinnen läßt.

### Verfahren der WO 98/08526

Für das erfindungsgemäße Verfahren können ebenso wie gemäß der WO 98/08526 der PMU als solcher, ein daraus durch Einengen gewonnenes Konzentrat oder ein daraus durch Membranfiltration gewonnenes Retentat eingesetzt werden. Der gesammelte Harn wird zunächst auf an sich bekannte Weise von Schleim- und Feststoffen befreit. Zweckmäßig läßt man Fest- und Schleimstoffe absetzen und trennt diese dann nach an sich bekannten Trennungsmethoden, beispielsweise Dekantieren, Separieren und/oder Filtrieren ab. So kann der PMU beispielsweise über eine an sich bekannte Trenneinrichtung, z.B. einen Separator, eine Filtrationsanlage oder einen Sedimentator geleitet werden. Als Trenneinrichtung kann z.B. ein Sandbett dienen, oder es können handelsübliche Separatoren, z.B. Düsen- oder Kammerseparatoren, eingesetzt werden. Gewünschtenfalls können auch eine Microfiltrationsanlage oder eine Ultrafiltrationsan-lage eingesetzt werden, bei deren Verwendung gleichzeitig eine weitgehende Keim- und Virenfreiheit des filtrierten PMU erreicht werden kann.

Gewünschtenfalls können dem Harn Konservierungsmittel, Germizide, Bakterizide und/oder Anthelmintika zugesetzt werden.

Sofern anstelle des PMU ein aufkonzentriertes PMU-Retentat eingesetzt werden soll, kann dieses aus dem PMU durch an sich bekannte Membranfiltration gewonnen werden. Der Feststoffgehalt des Retentates und dessen Zusammensetzung können je nach dem eingesetzten PMU und der zur Membranfiltration verwendeten Membran, beispielsweise deren Porenweite, sowie den Bedingungen der Filtration variieren. Beispielsweise kann bei Verwendung einer Nanofiltrationsmembran eine nahezu verlustfreie Konzentration des Oestrogengehaltes in dem PMU-Retentat bei gleichzeitiger Entfernung von bis zu 50 Gew.-% der niedermolekularen PMU-Inhaltsstoffe erreicht werden. Für das erfindungsgemäße Verfahren können PMU-Retentate eingesetzt werden, welche bis zu einem Verhältnis von ca. 1:10, beispielsweise einem Verhältnis von ca. 1:7 aufkonzentriert wurden und deren Volumen somit bis auf ca. 1/10, beispielsweise ca. 1/7, des ursprünglichen PMU-Volumens eingeengt sein kann.

Die in dem Verfahrensschritt a) einsetzbaren semipolaren polymeren Adsorberharze sind poröse organische nichtionische Polymere, welche im Gegensatz zu unpolaren hydrophoben polymeren Adsorberharzen eine intermediäre Polarität (= z.B. mit einem Dipolmoment der aktiven Oberfläche des Harzes im Bereich von 1,0 bis 3,0, insbesondere 1,5 bis 2,0, Debye) und eine etwas hydrophylere Struktur besitzen, beispielsweise Polycarbonsäureesterharze. Zweckmäßig werden makroporöse semipolare Harze mit vorzugsweise makroretikularer Struktur und mit durchschnittlichen Porendurchmessern im Bereich von 50 bis 150, vorzugsweise 70 bis 100, Angström und einer spezifischen Oberfläche im Bereich von 300 bis 900, vorzugsweise 400 bis 500, m²/g eingesetzt. Als besonders geeignet erweisen sich makroporöse quervernetzte aliphatische Polycarbonsäureesterharze, insbesondere quervernetzte Polyacrylesterharze wie z. B. Amberlite XAD-7^{R} der Fa. Rohm und Haas.

Die Adsorption der konjugierten Oestrogene an das semipolare Adsorberharz kann gemäß W0 98/08526 wie auch im vorliegenden erfindungsgemäßen Verfahren durch Kontaktierung des PMU oder dessen Retentats mit dem Adsorberharz erfolgen, indem die Harnflüssigkeit in einen das Adsorberharz enthaltenden Reaktor eingeführt und darin für eine zur Adsorption des Oestrogengehaltes ausreichende Zeit mit dem Adsorberharz in Kontakt gehalten wird. Nach erfolgter Adsorption der konjugierten Oestrogene an das semipolare Adsorberharz kann das mit dem Gemisch konjugierter Oestrogene beladene Adsorberharz von der übrigen Harnflüssigkeit auf an sich bekannte Weise abgetrennt werden. Zweckmäßigerweise kann die Harnflüssikgeit durch eine das Adsorberharz enthaltende Säule mit solcher Durchlaufgeschwindigkeit geleitet werden, daß die Kontaktzeit zur Adsorption des Oestrogengehaltes ausreicht. Geeignet sind beispielsweise Durchlaufgeschwindigkeiten, welche einem Durchlauf von 3 bis 10, vorzugsweise 5 bis 7, Vol.-Teilen PMU/1 Vol.-Teil Adsorberharz/Stunde entsprechen. Zweckmäßig kann die Durchlaufgeschwindigkeit der Harnflüssigkeit durch den Reaktor durch Arbeiten bei geringem Überdruck oder Unterdruck gesteuert werden. Die Menge an anzuwendendem semipolarem Adsorberharz kann je nach Art des verwendeten Adsorberharzes und Menge des Feststoff-Gehaltes in der Harnflüssigkeit variieren. Bei Verwendung von PMU kann beispielsweise ein Volumenteil Adsorberharz, z. B. quervernetztes aliphatisches Polycarbonsäureester-Adsorberharz, mit bis zu 80 Volumen-Teilen vorbehandeltem PMU beladen werden, ohne daß merkliche Mengen Oestrogen in der abfließenden Harnflüssigkgeit nachweisbar sind. Bei Verwendung eines PMU-Konzentrates oder PMU-Retentates reduziert sich die Beladungskapazität des Adsorberharzes natürlich in dem Maße, in dem diese aufkonzentriert sind. So kann beispielsweise 1 Volumenteil an quervernetztem aliphatischem Polycarbonsäureester-Adsorberharz mit einer 20 bis 80, vorzugsweise 30 bis 50, Volumenteilen PMU entsprechenden Menge Harnflüssigkeit beladen werden.

Das mit dem Gemisch konjugierter Oestrogene beladene semipolare Adsorberharz wird in Verfahrensschritt b) mit einem auf einen pH-Bereich von mindestens 12,0, insbesondere von 12,5 bis 14, vorzugsweise ca. 12,5 bis 13,5, eingestellten Waschwasser gewaschen. Als Waschwasser können wäßrige Lösungen von in der Harnflüssigkeit löslichen inerten basischen Substanzen, welche stark genug sind, um einen pH-Wert von mindestens 12,5 zu erreichen, eingesetzt werden. Als gegenüber dem semipolaren polymeren Adsorberharz inerte, wasserlösliche basische Substanzen eignen sich vorzugsweise wasserlösliche anorganische Basen wie Alkalimetall- oder Erdalkalimetallhydroxide, insbesondere Natriumhydroxid. Zweckmäßig enthält das Waschwasser nur etwa diejenige Menge an basischen Substanzen, welche zum Erreichen des gewünschten pH-Wertes, vorzugsweise ca. pH 13, benötigt wird. Die Menge an Waschwasser wird so gewählt, daß sie ausreicht, um phenolische Harninhaltsstoffe weitgehend zu entfernen, ohne daß dabei nennenswerte Mengen konjugierter Oestrogene mit ausgewaschen werden. Als zweckmäßig erweist sich beispielsweise die Verwendung von 2 bis 10, insbesondere 4 bis 6, Bettvolumen Waschflüssigkeit pro Bettvolumen Adsorberharz. Hierbei wird das Waschwasser zweckmäßigerweise durch einen das Adsorberharz enhaltenden Reaktor mit einer Durchflußgeschwindigkeit von 3 bis 10, vorzugsweise 5 bis 7, Vol.-Teilen Waschwasser /1 Vol.-Teil Adsorberharz/Stunde geleitet.

Im Verfahrensschritt c) wird anschließend das gewaschene mit dem Gemisch konjugierter Oestrogene beladene Adsorberharz mit einer zur Elution des Gemisches der konjugierten Oestrogene ausreichenden Menge einer Elutionsflüssigkeit behandelt und ein das natürliche Gemisch der konjugierten Oestrogene des PMU enthaltendes Eluat gewonnen. Die erfindungsgemäß verwendete Elutionsflüssigkeit stellt ein mit Wasser mischbares organisches Lösemittel aus der Gruppe mit Wasser mischbarer Ether, niederer Alkanole und niederer aliphatischer Ketone oder ein Gemisch aus einem solchen mit Wasser mischbaren Lösemittel und gegebenenfalls alkalisch gestelltem Wasser dar. Als Etherbestandteile der Elutionsflüssigkeit eignen sich mit Wasser mischbare cyclische Ether wie Tetrahydrofuran oder Dioxan, aber auch mit Wasser mischbare offenkettige Ether wie beispielsweise Ethylenglycoldimethylether (= Monoglyme), Diethylenglycoldimethylether (= Diglyme) oder Ethyloxyethyloxyethanol (= Carbitol). Als niedere Alkanole eignen sich mit Wasser mischbare Alkylalkohole mit 1 - 4, vorzugsweise 1 - 3, Kohlenstoffatomen, insbesondere Ethanol oder Isopropanol. Als niedere aliphatische Ketone eignen sich mit Wasser mischbare Ketone mit 3 - 5 Kohlenstoffatomen, insbesondere Aceton. Als besonders günstig erweisen sich Elutionsflüssigkeiten, in denen das organische Lösemittel Ethanol ist. Zweckmäßig werden als Elutionsflüssigkeiten Gemische aus einem der vorgenannten mit Wasser mischbaren organischen Lösemittel und gegebenenfalls alkalisch eingestelltem Wasser eingesetzt. Der pH-Wert derartiger wasserhaltiger Elutionsmittel liegt im neutralen bis alkalischen Bereich bis zu pH 13 und kann vorteilhaft ca. 10 bis 12 betragen. Als Lösemittelkomponente in der wasserhaltigen Elutionsflüssigkeit wird ein in dem eingesetzten pH-Bereich stabiles Lösemittel ausgewählt. In wasserhaltigen alkalischen Elutionsflüssigkeiten mit pH-Werten von ca. 10 bis 12 eignen sich als Lösemittelkomponente niedere Alkanole, vorzugsweise Ethanol. Der gewünschte pH-Wert der wasserhaltigen Elutionsmittel wird durch Zugabe einer entsprechenden Menge einer wasserlöslichen inerten basischen Substanz, vorzugsweise einer anorganischen Base, beispielsweise eines Alkalimetall- oder Erdalkalimetallhydroxides, insbesondere Natriumhydroxid, eingestellt. In wasserhaltigen Elutionsflüssigkeiten kann ein Volumenverhältnis von mit Wasser mischbarem organischem Lösemittel zu Wasser im Bereich von 40:60 bis 20:80, vorzugsweise ca. 30:70 vorliegen. Die eingesetzte Menge an Elutionsmittel kann ca. 3 bis 10, insbesondere ca. 4 bis 6, Bettvolumen pro Bettvolumen Adsorberharz betragen. Zweckmäßigerweise wird die Elutionsflüssigkeit durch einen das mit dem Oestrogengemisch beladene Adsorberharz enthaltenden Reaktor mit einer solchen Durchlaufgeschwindigkeit geleitet, daß die Kontaktzeit zur vollständigen Elution des Gemisches konjugierter Oestrogene ausreicht. Bei Verwendung eines Gemisches von Ethanol mit Wasser im Vol.-Verhältnis 30:70 eignen sich beispielsweise Durchlaufgeschwindigkeiten von 3 bis 10, vorzugsweise 5 bis 7, Vol.-Teilen Elutionsflüssigkeit pro 1 Vol.-Teil Adsorberharz pro Stunde. Zweckmäßig wird die Elution in einem Temperaturbereich von Raumtemperatur bis ca. 60 °C, vorzugsweise bei ca. 40 bis 50 °C, durchgeführt. Gewünschtenfalls wird die Durchlaufgeschwindigkeit durch Arbeiten bei leicht erhöhtem Druck, z. B. bei einem Überdruck von bis zu 0,2 bar, reguliert und das Eluat in mehreren Fraktionen aufgefangen. Die Gehalte der einzelnen Eluatfraktionen an konjugierten Oestrogenen und phenolischen Harninhaltsstoffen wie Kresolen und HPMF können auf an sich bekannte Weise durch Hochleistungsflüssigkeits-Chromatographie (= high performance liquid chromatography, abgekürzt als "HPLC") bestimmt werden.

Bei der Elution wird zunächst eine schwachgefärbte bis farblose, praktisch oestrogenfreie Vorlauffraktion erhalten, deren Menge im allgemeinen ca. einem Bettvolumen entspricht. Die Hauptmenge der konjugierten Oestrogene, beispielsweise zwischen 80 und 99 % der in dem Ausgangs-PMU vorliegenden konjugierten Oestrogene, befindet sich in den nachfolgenden dunkelgelb-braun gefärbten Haupteluatfraktionen, deren Menge im allgemeinen 2 bis 4 Bettvolumen umfaßt. In den nachfolgenden Nachlauffraktionen sind im allgemeinen nur noch Spuren an konjugierten Oestrogenen enthalten. Sofern Nachfolgefraktionen erhalten werden, welche noch einem Gehalt an konjugierten Oestrogenen von über 10 Gew.-% bezogen auf Trockensubstanz und weniger als 0,6 Gew.-% bezogen auf Trockensubstanz an Kresolen und HPMF enthalten, können diese mit dem oestrogenreichen Haupteluat zur Weiterverarbeitung vereinigt werden.

### Beispiel 1 (Vergleichsbeispiel):

### a) Adsorption des Oestrogengehaltes des PMU an ein semipolares Polyacrylester-Adsorberharz

Es wird eine Säule von 220 mm Höhe mit einem Durchmesser von 40 mm mit 300 ml eines in Wasser gequollenen semipolaren Polyacrylester-Adsorberharz (= Amberlite XAD-7 der Fa. Rohm und Haas, Korngröße 0,3 bis 1,2 mm, Dipolmoment 1,8 Debye, durchschnittlicher Porendurchmesser 80 Angström, spezifische Oberfläche ca. 450 m²/g trocken) gefüllt. 10,5 l (= 35 Bettvolumen) eines durch eine Ultrafiltrationsanlage filtrierten PMU (mittels HPLC bestimmte Gehalte an Oestronsulfatsalz und Kresol siehe nachfolgende Beispielstabelle) werden bei Raumtemperatur mit einer Durchflussgeschwindigkeit von durchschnittlich 24 ml/min (= 4,8 Bettvolumen pro Stunde) durch die Säule geleitet. Der Oestrogengehalt des PMU ist vollständig auf der so beladenen semipolaren Adsorberharz-Säule adsorbiert. Die ablaufende Harnflüssigkeit wird mittels HPLC auf ihren Gehalt an konjugierten Oestrogenen (berechnet als Oestronsulfatsalz) untersucht und erweist sich als praktisch oestrogenfrei. Der Ablauf wird verworfen.

### b) Waschen der beladenen Adsorberharz-Säule

Die beladene Adsorberharz-Säule wird mit 1,5 1 einer wässrigen Natriumhydroxidlösung mit einem pH-Wert von 13 gewaschen. Hierzu wird das alkalische Waschwasser mit einer Durchflussgeschwindigkeit von durchschnittlich 24,6 ml/min (= 4,9 Bettvolumen pro Stunde) durch die Säule geleitet. Die ablaufende Waschflüssigkeit wird mittels HPLC auf ihren Gehalt an Oestronsulfatsalz und Kresol untersucht. Die Untersuchung zeigt, dass während der Waschphase weniger als 5 % der gesamten auf die Säule aufgegebenen Oestrogene ausgewaschen wird.

### c) Desorption der konjugierten Oestrogene von der gewaschenen Adsorberharz-Säule

1,5 1 der Elutionsflüssigkeit (Ethanol/Wasser 30:70) werden mit einer Fliessgeschwindigkeit von 25 ml/min (= 5,0 Bettvolumen pro Stunde) durch die auf die in der Beispieltabelle angegebene Elutionstemperatur vorgeheizte Säule geleitet. Das ablaufende Eluat wird in 5 Fraktionen aufgefangen. Die Fraktionen betragen jeweils 300 ml (= 1 Bettvolumen) und werden mittels HPLC auf ihren Gehalt an Oestronsulfatsalz und Kresol untersucht.

Die erste Fraktion enthält nur Spuren an Oestrogensulfatsalz. Das Eluat erscheint farblos bis schwach gelb gefärbt. In den nachfolgenden Fraktionen 2 bis 4 sind dann ca. 80 bis 98 % der gesamten auf der Säule adsorbierten Menge konjugierter Oestrogene enthalten. Die Eluate haben hier einen intensiven dunkelbraunen Farbton. Die letzte Fraktion enthält nur noch eine geringe Menge an Oestrogensulfatsalz, was auch deutlich an der Abnahme der Farbintensität sichtbar ist.

Für die die Hauptmenge der konjugierten Oestrogene enthaltenden Fraktionen sind in der nachfolgenden Tabelle jeweils der TS-Gehalt in Gew.-% und die durch HPLC bestimmten Gehalte an Oestronsulfatsalz und Kresol angegeben. Diese Fraktionen stellen für eine galenische Weiterverarbeitung geeignete Extrakte dar.

### d) Regenerierung der Adsorberharz-Säule

Zur Regenerierung wird die Säule zunächst mit 600 ml eines auf pH 12 eingestellten 50 % Ethanol enthaltenden Ethanol/Wasser-Gemisches, dann mit 600 ml eines auf pH 12 eingestellten 70 % Ethanol enthaltenden Ethanol/Wasser-Gemisches, anschließend mit 600 ml 10-%iger wässriger Natriumcitratlösung und schließlich mit 600 ml destilliertem Wasser gewaschen. Die gesamte Regeneration erfolgt bei einer Temperatur von 45 °C. Die Säule kann mehrmals, beispielsweise bis zu 40-mal, beladen und wieder regeneriert werden.

### Beispiel 2 (Erfindungsgemäßes Verfahren):

Beispiel 1 wurde mit der erfindungsgemäßen Zwischenwäsche zwischen den Verfahrensschritten b) und c) wiederholt.

### Zwischenwäsche

Die beladene Adsorberharz-Säule wird mit 900 ml Wasser gewaschen. Hierzu wird das neutrale Waschwasser mit einer Durchflussgeschwindigkeit von durchschnittlich 25,0 ml/min (= 5,0 Bettvolumen pro Stunde) durch die Säule geleitet.

Die ablaufende Waschflüssigkeit wird mittels HPLC auf ihren Gehalt an Oestronsulfatsalz und Kresol untersucht. Die Untersuchung zeigt, dass während dieser Waschphase nur ein geringer Teil (maximal nur etwa einige Prozent) der gesamten auf die Säule aufgegebenen Oestrogene ausgewaschen wird. Erfindungsgemäß reduziert sich aber bei Durchführung des Erfindungsgemäßen Verfahrens auch der Anteil an freien Oestrogenen (siehe Beispiel 4).

**Tabelle I:**

| Ergebnisse des Vergleichsbeispiels 1 und des erfindungsgemäßen Beispiels 2 | | | | | | |
|---|---|---|---|---|---|---|
| **Beispiel Nr.** | | | **1** | | **2** | |
| **Verfahrensschritt a):** | | | | | | |
| Ausgangs-PMU | | | | | | |
| Estron | | mg/l | 86,4 | | 92,3 | |
| Equilin | | mg/l | 57,0 | | 57,0 | |
| Kresol | | mg/l | 1191,1 | | 877,6 | |

| **Verfahrensschritt b):** | | | | | | |
|---|---|---|---|---|---|---|
| Waschwasser = wässrige NaOH-Lösung | | | pH 13 | | pH 13 | |

| **Zwischenwäsche:** | | | | | | |
|---|---|---|---|---|---|---|
| Waschwasser = Wasser | | | | | | |
| Waschfraktion 1: | | | | | | |
| Estron | | mg/l | | | 0,0 | |
| Equilin | | mg/l | | | 20,9 | |
| Kresol | | mg/l | Vergleich: | | 18,0 | |
| Waschfraktion 2: | | | Zwischenwäsche | | | |
| Estron | | mg/l | nicht | | 125,0 | |
| Equilin | | mg/l | | | 0,0 | |
| Kresol | | mg/l | durchgeführt | | 0,0 | |
| Waschfraktion 3: | | | | | | |
| Estron | | mg/l | | | 290,6 | |
| Equilin | | mg/l | | | 138,0 | |
| Kresol | | mg/l | | | 31,5 | |
| | | | | | | |

| **Verfahrensschritt c):** | | | | | | |
|---|---|---|---|---|---|---|
| Elutionsflüssigkeit | | | Ethanol/Wasser 30 : 70 | | Ethanol/Wasser 30 : 70 | |
| Elutionstemperatur | | | 45°C | | 45 °C | |

| Eluatfraktion 1: | | | | | | |
|---|---|---|---|---|---|---|
| | Gewichts-% TS | | 4,3 | | 0,2 | |
| Estron | | mg/l (Gew.-% TS) | 0,0 | (0,00) | 342,7 | (17,14) |
| Equilin | | mg/l (Gew.-% TS) | 24,8 | (0,06) | 182,6 | (9,13) |

| Eluatfraktion 2: | | | | | | |
|---|---|---|---|---|---|---|
| | Gewichts-% TS | | 1,9 | | 0,9 | |
| Estron | | mg/l (Gew.-% TS) | 2788,6 | (14,68) | 2053,5 | (22,81) |
| Equilin | | mg/l (Gew.-% TS) | 1528,0 | (8,04) | 1120,1 | (12,45) |

| Eluatfraktion 3: | | | | | | |
|---|---|---|---|---|---|---|
| | Gewichts-%-TS | | 0,2 | | 0,2 | |
| Estron | | mg/l (Gew.-% TS) | 193,2 | (9,66) | 38,9 | (1,95) |
| Equilin | | mg/l (Gew.-% TS) | 99,8 | (4,99) | 19,8 | (0,99) |

| Eluatfraktion 4: | | | | | | |
|---|---|---|---|---|---|---|
| | Gewichts-%-TS | | 0,1 | | 0,1 | |
| Estron | | mg/l (Gew.-% TS) | 7,1 | (0,71) | 0,0 | (0,00) |
| Equilin | | mg/l (Gew.-% TS) | 9,3 | (0,93) | 0,0 | (0,00) |

### Beispiel 3 (erfindungsgemäßes Verfahren mit Kühlung):

### Reduzierung der Hormonverluste während der Neutralen Wäsche

Es wird wie in den Beispielen 1 und 2, jedoch unter Kühlung gearbeitet. Die Kühlung dient zur Minimierung von Hormonverlusten während der Zwischenwäsche. Wird die Zwischenwäsche statt bei Raumtemperatur mit Eiswasser durchgeführt, so können die Hormonverlusten um bis zu ca. 50 % gesenkt werden, insbesondere wenn im Verfahren die Temperatur tatsächlich 0 °C bzw. annähernd 0 °C beträgt. Hierbei empfiehlt es sich, nicht nur Eiswasser aufzugeben, welches sich aufgrund des mit Raumtemperatur vorliegenden Säuleninhaltes relativ schnell erwärmen kann, sondern es ist vielmehr die gesamte Säule auf 0 °C zu kühlen.

### Versuchsdurchführung

Die für die Zwischenwäsche erforderliche Säulentemperatur von 0 °C wurde durch eine Abkühlung während der vorgehenden basischen Wäsche erreicht, d.h. bereits die basische Wäsche lief bei 0 °C. Die Aufgabe von 5 Bettvolumen mit 0 °C bei der Basischen Wäsche war dabei ausreichend, um den Säuleninhalt von Raumtemperatur auf 0 °C abzukühlen. Die sich anschließende Zwischenwäsche konnte dann bei 0 °C ablaufen. Neben der Minimierung der Hormonverluste wurde auch der mögliche Temperatureinfluss auf die Kresolabtrennung während der basischen Wäsche mit untersucht.

Zum direkten Vergleich wurden zwei Versuche durchgeführt:

### Beispiel 3a:

Basische Wäsche und Zwischenwäsche bei Raumtemperatur.

### Beispiel 3b:

Basische Wäsche und Zwischenwäsche Wäsche bei 0 °C.

Alle anderen Schritte erfolgten im Standardverfahren wie oben für das in der WO 98/08526 beschriebene Verfahren oder in den Beispielen 1 und 2 angegeben.

### Versuchsergebnisse

Die Tabelle III gibt die Versuchsparameter und Versuchsergebnisse bei Raumtemperatur und mit Temperierung auf 0 °C wieder.

Die Versuchsergebnisse lassen deutlich erkennen, dass
a) die basische Wäsche, d.h. die Kresolentfernung, durch die Temperaturabsenkung auf 0 °C nicht beeinträchtigt wird. Die Abtrennung von Kresol verläuft quantitativ. Die Tabelle III gibt einen Überblick.
b) die Hormonverluste während der Zwischenwäsche durch die Temperaturerniedrigung um ca. 50 % reduziert werden. Die Tabelle II gibt einen Überblick.

**Tabelle II Hormonverluste und deren Minimierung durch Zwischenwäsche bei 0 °C**

| **Beispiel Nr.** | **3a** | **3b** |
|---|---|---|
| | **Raumtemperatur** | **Eiswasser** |
| | **(T = ca. 25 °C)** | **(T = ca. 0·°C)** |
| **Estron** | 2,7 Gew.-% | 1,8 Gew.-% |
| **Equilin** | 3,4 Gew.-% | 1,4 Gew.-% |

Die Aufgabe von auf etwa 0 °C temperierter Natronlauge während der basischen Wäsche bietet damit eine einfache Möglichkeit, ohne Beeinträchtigung der Kresolabtrennung die Hormonverluste der Zwischenwäsche durch Arbeiten bei etwa 0 °C ebenfalls zu senken.

Die Absenkung der Temperatur während der Zwischenwäsche auf ca. 0 °C ermöglicht eine Minimierung, d.h. Verminderung der durch die zusätzliche Zwischenwäsche unvermeidlichen Hormonverluste um bis zu etwa 50 %.

Die Säulentemperierung während der Zwischenwäsche kann auf einfache Weise erreicht werden, wenn die vor der Zwischenwäsche durchgeführte basische Wäsche ebenfalls bei ca. 0 °C abläuft. Die bei der basischen Wäsche stattfindende Kresolabtrennung wird durch die Temperaturerniedrigung nicht negativ beeinflußt.

**Tabelle III**

| Durchführung des erfindungsgemäßen Verfahrens bei Raumtemperatur und unter Kühlung | | | | | | |
|---|---|---|---|---|---|---|
| **Beispiel Nr.** | | | **3a** | | **3b** | |
| | | | **Raumtemperatur** | | **Eiswasser** | |
| | | | **(T = ca. 25 °C)** | | **(T = ca. 0 °C)** | |
| **Verfahrensschritt a):** | | | | | | |
| Ausgangs-PMU | | | | | | |
| Estron | | mg/l | 64,9 | | 65,4 | |
| Equilin | | mg/l | 57,9 | | 57,6 | |
| Kresol | | mg/l | 329,0 | | 333,0 | |

| **Verfahrensschritt b):** | | | | | | |
|---|---|---|---|---|---|---|
| Waschwasser = wässrige NaOH-Lösung | | | pH 13 | | pH 13 | |

| Waschfraktion 1: | | | | | | |
|---|---|---|---|---|---|---|
| Estron | | mg/l | 4,4 | | 1,7 | |
| Equilin | | mg/l | 0,0 | | 1,6 | |
| Kresol | | mg/l | 55,1 | | 51,6 | |

| Waschfraktion 2: | | | | | | |
|---|---|---|---|---|---|---|
| Estron | | mg/l | 28,8 | | 27,9 | |
| Equilin | | mg/l | 31,5 | | 24,8 | |
| Kresol | | mg/l | 6942,5 | | 7011,0 | |

| Waschfraktion 3: | | | | | | |
|---|---|---|---|---|---|---|
| Estron | | mg/l | 0,0 | | 0,0 | |
| Equilin | | mg/l | 11,5 | | 18,3 | |
| Kresol | | mg/l | 2215,2 | | 2774,0 | |

| Waschfraktion 4: | | | | | | |
|---|---|---|---|---|---|---|
| Estron | | mg/l | 0,0 | | 0,6 | |
| Equilin | | mg/l | 0,0 | | 3,2 | |
| Kresol | | mg/l | 280,6 | | 268,6 | |

| Waschfraktion 5: | | | | | | |
|---|---|---|---|---|---|---|
| Estron | | mg/l | 0,0 | | 0,0 | |
| Equilin | | mg/l | 0,0 | | 0,0 | |
| Kresol | | mg/l | 76,6 | | 44,1 | |

| **Hormonverluste Waschfraktion 1 - 5** | | | | | | |
|---|---|---|---|---|---|---|
| Estron | | | 1,5 % | | 1,3 % | |
| Equilin | | | 2,1 % | | 2,4 % | |

| **Zwischenwäsche:** | | | | | | |
|---|---|---|---|---|---|---|
| Waschwasser = Wasser | | | | | | |
| Waschfraktion 1: | | | | | | |
| Estron | | mg/l | 0,0 | | 0,0 | |
| Equilin | | mg/l | 0,0 | | 0,0 | |
| Kresol | | mg/l | 22,8 | | 19,4 | |

| Waschfraktion 2: | | | | | | |
|---|---|---|---|---|---|---|
| Estron | | mg/l | 13,3 | | 8,2 | |
| Equilin | | mg/l | 9,7 | | 5,9 | |
| Kresol | | mg/l | 33,2 | | 11,8 | |

| Waschfraktion 3: | | | | | | |
|---|---|---|---|---|---|---|
| Estron | | mg/l | 48,0 | | 32,0 | |
| Equilin | | mg/l | 58,3 | | 23,3 | |
| Kresol | | mg/l | 76,0 | | 0,0 | |

| **Hormonverluste Waschfraktion 1 - 3** | | | | | | |
|---|---|---|---|---|---|---|
| Estron | | | 2,7 % | | 1,8 % | |
| Equilin | | | 3,4 % | | 1,4 % | |
| | | | | | | |

| **Verfahrensschritt c):** | | | | | | |
|---|---|---|---|---|---|---|
| Elutionsflüssigkeit | | | Ethanol/Wasser | | Ethanol/Wasser | |
| | | | 30 : 70 | | 30 : 70 | |
| Elutionstemperatur 45 °C | | | | | 45 °C | |

| Eluatfraktion 1: | | | | | | |
|---|---|---|---|---|---|---|
| | Gewichts-% TS | | 0,2 | | 0,2 | |
| Estron | | mg/l (Gew.-% TS) | 76,0 | (3,80) | 58,0 | (2,90) |
| Equilin | | mg/l (Gew.-% TS) | 65,8 | (3,29) | 48,2 | (2,41) |

| Eluatfraktion 2: | | | | | | |
|---|---|---|---|---|---|---|
| | Gewichts-% TS | | 1,0 | | 0,9 | |
| Estron | | mg/l (Gew.-% TS) | 1237,0 | (12,37) | 1145,6 | (12,73) |
| Equilin | | mg/l (Gew.-% TS) | 1093,8 | (10,94) | 1135,0 | (12,61) |

| Eluatfraktion 3: | | | | | | |
|---|---|---|---|---|---|---|
| | Gewichts-%-TS | | 0,4 | | 0,5 | |
| Estron | | mg/l (Gew.-% TS) | 607,8 | (15,20) | 840,0 | (16,80) |
| Equilin | | mg/l (Gew.-% TS) | 524,7 | (13,12) | 748,4 | (14,97) |

| Eluatfraktion 4: | | | | | | |
|---|---|---|---|---|---|---|
| | Gewichts-%-TS | | 0,1 | | 0,1 | |
| Estron | | mg/l (Gew.-% TS) | 84,6 | (8,46) | 144,6 | (14,46) |
| Equilin | | mg/l (Gew.-% TS) | 58,2 | (5,82) | 107,4 | (10,74) |
| Kresol | | mg/l (Gew.-% TS) | 0,0 | (0,00) | 0,0 | (0,00) |

| | | | | | | |
|---|---|---|---|---|---|---|
| Eluatfraktion 5: | | | 0,1 | | 0,1 | |
| | Gewichts-%-TS | | 21,1 | (2,11) | 34,3 | (3,43) |
| Estron | | mg/l (Gew.-% TS) | 7,5 | (0,75) | 17,9 | (1,79) |
| Equilin | | mg/l (Gew.-% TS) | | | | |

| | | | | | | |
|---|---|---|---|---|---|---|
| Eluatfraktion 6: | | | 9,0 | | 15,2 | |
| Estron | | mg/l | 0,0 | | 3,4 | |
| Equilin | | mg/l | | | | |

### Beispiel 4 (Verminderung des Gehaltes an freien Hormonen):

Gemäß den Verfahrensweisen der vorhergehenden Beispiele 1 bis 3 wurde untersucht, wie sich der Gehalt an freien Hormonen bei Anwendung des erfindungsgemäßen Verfahrens vermindert. Die Ergebnisse sind in den nachfolgenden Tabelle IV a (Vergleichsversuche ohne Zwischenwäsche)und IV b (erfindungsgemäße Versuche mit Zwischenwäsche) dargestellt.

### Tabelle IV Minimierung der freien Hormone:

**Tabelle IV a Vergleichsversuche ohne Zwischenwäsche**

| | **Gehalt an freien Estrogenen im Konzentrat** | **Gesamtgehalt an konjugierten Estrogenen bezogen auf den Trockensubstanzgehalt im Konzentrat** |
|---|---|---|
| | **(Gew.-%)** | **(Gew.-%)** |
| **Versuch 1** | 3,21 | 3,2 |
| **Versuch 2** | 3,67 | 4,4 |
| **Versuch 3** | 2,38 | 9,2 |
| **Versuch 4** | 4,14 | 12,2 |
| **Versuch 5** | 4,03 | 9,9 |

**Tabelle IV b Versuche mit Zwischenwäsche (erfindungsgemäß)**

| | **Gehalt an freien Estrogenen im Konzentrat** | **Gesamtgehalt an konjugierten Estrogenen bezogen auf den Trockensubstanzgehalt im Konzentrat** |
|---|---|---|
| | **(Gew.-%)** | **(Gew.-%)** |
| **Versuch 6** | 0,0 | 25,2 |
| **Versuch 7** | 0,77 | 17,2 |
| **Versuch 8** | 0,35 | 23,9 |
| **Versuch 9** | 0,20 | 29,3 |
| **Versuch 10** | 0,0 | 27,7 |

## Patentansprüche

1. Verfahren zur Gewinnung eines an phenolischen Harninhaltsstoffen abgereicherten natürlichen Gemisches konjugierter Oestrogene aus dem Harn trächtiger Stuten, bei dem man
a) eine Harnflüssigkeit, welche gegebenenfalls eine von Schleim- und Feststoffen befreite Harnfüssigkeit, ein eingeengtes Konzentrat dieser Harnflüssigkeit oder ein durch Membranfiltration dieser Harnflüssigkeit erhaltenes eingeengtes Harnretentat darstellt, mit einer zur Adsorption des in der Harnflüssigkeit enthaltenen Gemisches konjugierter Oestrogene ausreichenden Menge eines polymeren Adsorberharzes kontaktiert und ein mit dem Gemisch konjugierter Oestrogene beladenes polymeres Adsorberharz von der übrigen Harnflüssigkeit abtrennt,
b) das mit dem Gemisch konjugierter Oestrogene beladene polymere Adsorberharz mit einem auf einen pH-Bereich von mindestens 12,0 eingestellten Waschwasser wäscht, und
c) das gewaschene Adsorberharz mit einer zur Desorption des daran adsorbierten Gemisches konjugierter Oestrogene ausreichenden Menge einer Elutionsflüssigkeit, welche ein mit Wasser mischbares organisches Lösemittel aus der Gruppe mit Wasser mischbarer Ether, niederer Alkanole und niederer aliphatischer Ketone oder ein Gemisch aus dem mit Wasser mischbaren organischen Lösemittel und gegebenenfalls alkalisch eingestelltem Wasser darstellt, kontaktiert und ein das natürliche Gemisch konjugierter Oestrogene enthaltendes Eluat von dem Adsorberharz abtrennt und gegebenenfalls einengt,
**dadurch gekennzeichnet, dass**
zwischen den Verfahrensschritten b) und c) eine Zwischenwäsche durchgeführt wird, bei der das mit dem Gemisch konjugierter Oestrogene beladene polymere Adsorberharz mit Wasser gewaschen wird.

2. Verfahren nach Anspruch 1, **dadurch gekennzeichnet, dass** man die Zwischenwäsche bei Temperaturen unterhalb Raumtemperatur, insbesondere bei Temperaturen zwischen 0 °C und 10 °C, vorzugsweise bei Temperaturen nahe oberhalb 0 °C, durchführt.

3. Verfahren nach einem der vorstehenden Ansprüche, **dadurch gekennzeichnet, dass** das für die Zwischenwäsche verwendete Waschwasser auf Temperaturen unterhalb Raumtemperatur, insbesondere auf Temperaturen zwischen 0 °C und 10 °C, vorzugsweise auf Temperaturen nahe oberhalb 0 °C, vorgekühlt ist.

4. Verfahren nach Anspruch 1, **dadurch gekennzeichnet, dass** das im Verfahrensschritt b) verwendete, alkalische gestellte Waschwasser auf Temperaturen unterhalb Raumtemperatur, insbesondere auf Temperaturen zwischen 0 °C und 10 °C, vorzugsweise auf Temperaturen nahe oberhalb 0 °C, vorgekühlt ist.

5. Verfahren nach einem der vorstehenden Ansprüche, **dadurch gekennzeichnet, dass** die Zwischenwäsche und der vorhergehende Verfahrensschritt b) bei Temperaturen unterhalb Raumtemperatur, insbesondere bei Temperaturen zwischen 0 °C und 10 °C, vorzugsweise bei Temperaturen nahe oberhalb 0 °C, durchgeführt werden.

6. Verfahren nach einem der vorstehenden Ansprüche, **dadurch gekennzeichnet, dass** die verwendeten Vorrichtungen wie Reaktoren zur Aufnahme des polymere Adsorberharzes oder dieses bereits enthaltende Reaktoren und/oder der eingesetzte PMU entsprechend auf Temperaturen unterhalb Raumtemperatur, insbesondere auf Temperaturen zwischen 0 °C und 10 °C, vorzugsweise auf Temperaturen nahe oberhalb 0 °C, vorgekühlt eingesetzt werden.

7. Verfahren nach einem der vorstehenden Ansprüche, **dadurch gekennzeichnet, dass** als polymere Adsorberharze semipolare Adsorberharze, vorzugsweise nicht-ionische semipolare Adsorberharze, verwendet werden.

## Claims

1. A method for obtaining a natural mixture, depleted in phenolic urine contents, of conjugated oestrogens from pregnant mares' urine, in which
a) a urine, which optionally represents a urine freed of mucilaginous substances and solids, a reduced concentrate of this urine or a reduced urine retentate obtained by membrane filtration of this urine, is contacted with an amount of a polymeric adsorption resin sufficient for the adsorption of the mixture of conjugated oestrogens contained in the urine and a polymeric adsorption resin laden with the mixture of conjugated oestrogens is separated off from the rest of the urine,
b) the polymeric adsorption resin laden with the mixture of conjugated oestrogens is washed with a washing water which has been set to a pH range of at least 12.0, and
c) the washed adsorption resin is contacted with an amount of an elution liquid, sufficient for the desorption of the mixture of conjugated oestrogens adsorbed thereon, which represents a water-miscible organic solvent from the group of water-miscible ethers, lower alkanols and lower aliphatic ketones or a mixture of the water-miscible organic solvent and water which has optionally been rendered alkaline, and an eluate containing the natural mixture of conjugated oestrogens is separated off from the adsorption resin and optionally reduced,
**characterised in that**
between process steps b) and c) an intermediate washing operation is carried out, in which the polymeric adsorption resin laden with the mixture of conjugated oestrogens is washed with water.

2. A method according to Claim 1, **characterised in that** the intermediate washing is carried out at temperatures below room temperature, particularly at temperatures between 0°C and 10°C, preferably at temperatures close to but above 0°C.

3. A method according to one of the preceding claims, **characterised in that** the washing water used for the intermediate washing operation is precooled to temperatures below room temperature, in particular to temperatures between 0°C and 10°C, preferably to temperatures close to but above 0°C.

4. A method according to Claim 1, **characterised in that** the washing water which has been rendered alkaline which is used in process step b) is precooled to temperatures below room temperature, in particular to temperatures between 0°C and 10°C, preferably to temperatures close to but above 0°C.

5. A method according to one of the preceding claims, **characterised in that** the intermediate washing and the preceding process step b) are carried out at temperatures below room temperature, in particular at temperatures between 0°C and 10°C, preferably at temperatures close to but above 0°C.

6. A method according to one of the preceding claims, **characterised in that** the devices used, such as reactors for holding the polymeric adsorption resin or reactors already containing such and/or the PMU used are used precooled accordingly to temperatures below room temperature, in particular to temperatures between 0°C and 10°C, preferably to temperatures close to but above 0°C.

7. A method according to one of the preceding claims, **characterised in that** semipolar adsorption resins, preferably non-ionic semipolar adsorption resins, are used as polymeric adsorption resins.

## Revendications

1. Procédé d'obtention d'un mélange naturel d'oestrogènes conjugués, appauvri en constituants d'urine phénoliques à partir d'urine de juments gravides, dans lequel
a) on met en contact un liquide urinaire, qui est éventuellement un liquide urinaire débarrassé des matières mucilagineuses et solides, un produit concentré à une plus faible quantité de ce liquide urinaire ou un rétentat d'urine concentré obtenu par filtration sur membrane de ce liquide urinaire, avec une résine adsorbante polymère en quantité suffisante pour l'adsorption du mélange d'oestrogènes conjugués contenu dans le liquide urinaire, et on sépare du reste du liquide urinaire, une résine adsorbante polymère chargée en mélange d'oestrogènes conjugués,
b) on lave la résine adsorbante polymère chargée en mélange d'oestrogènes conjugués avec une eau de lavage dont la plage de pH est ajustée à au moins 12,0, et
c) on met en contact la résine adsorbante lavée avec un liquide d'élution en quantité suffisante pour la désorption du mélange d'oestrogènes conjugués qui y est adsorbé, le liquide d'élution étant un solvant organique miscible à l'eau appartenant au groupe des éthers, des alcanols inférieurs et des cétones aliphatiques inférieures miscibles à l'eau ou un mélange constitué du solvant organique miscible à l'eau et éventuellement d'eau rendue alcaline, et on sépare de la résine adsorbante un produit d'élution contenant le mélange naturel d'oestrogènes conjugués et éventuellement on le concentre,
**caractérisé en ce qu'**on effectue un lavage intermédiaire entre les étapes b) et c) du procédé, au cours duquel la résine adsorbante chargée en mélange d'oestrogènes conjugués est lavée à l'eau.

2. Procédé selon la revendication 1, **caractérisé en ce qu'**on effectue le lavage intermédiaire à des températures inférieures à la température ambiante, en particulier à des températures comprises entre 0 °C et 10 °C, de préférence à des températures légèrement au-dessus de 0 °C.

3. Procédé selon l'une des revendications précédentes, **caractérisé en ce que** l'eau de lavage utilisée pour le lavage intermédiaire est préalablement refroidie à des températures inférieures à la température ambiante, en particulier à des températures comprises entre 0 °C et 10 °C, de préférence à des températures légèrement au-dessus de 0 °C.

4. Procédé selon la revendication 1, **caractérisé en ce que** l'eau de lavage rendue alcaline, utilisée dans l'étape b) du procédé, est préalablement refroidie à des températures inférieures à la température ambiante, en particulier à des températures comprises entre 0 °C et 10 °C, de préférence à des températures légèrement au-dessus de 0 °C.

5. Procédé selon l'une des revendications précédentes, **caractérisé en ce que** le lavage intermédiaire et l'étape b) précédente du procédé sont réalisés à des températures inférieures à la température ambiante, en particulier à des températures comprises entre 0 °C et 10 °C, de préférence à des températures légèrement au-dessus de 0 °C.

6. Procédé selon l'une des revendications précédentes, **caractérisé en ce que** les dispositifs utilisés tels que les réacteurs destinés à recevoir la résine adsorbante polymère ou les réacteurs la contenant déjà et/ou l'urine de jument gravide mises en oeuvre sont, en conséquence, utilisés en étant préalablement refroidis à des températures inférieures à la température ambiante, en particulier à des températures comprises entre 0 °C et 10 °C, de préférence à des températures légèrement au-dessus de 0 °C.

7. Procédé selon l'une des revendications précédentes, **caractérisé en ce que** l'on utilise, en tant que résines adsorbantes polymères, des résines adsorbantes semi-polaires, de préférence des résines adsorbantes semi-polaires non ioniques.
